# EUROPEAN PATENT SPECIFICATION

(11) **EP 4 267 696 B1**
(45) Date of publication and mention of the grant of the patent: **12.03.2025**
(21) Application number: 21843929.7
(22) Date of filing: 21.12.2021
(51) Int. Cl.: C10B 53/07, C10G 1/10, C10L 9/08

(54) **PROCESS FOR THE DEPOLYMERIZATION OF PLASTIC WASTE MATERIAL**
VERFAHREN ZUR DEPOLYMERISATION VON KUNSTSTOFFABFALLMATERIAL
PROCÉDÉ DE DÉPOLYMÉRISATION DE DÉCHETS PLASTIQUES

(30) Priority: 22.12.2020 EP 20216344
(43) Date of publication of application: 01.11.2023
(73) Proprietor: Basell Poliolefine Italia S.r.l., 20121 Milano (IT)
(72) Inventor: ARICH DE FINETTI, Nicolò, 44122 Ferrara (IT); BRITA, Diego, 44122 Ferrara (IT); MAZZUCCO, Antonio, 44122 Ferrara (IT)
(74) Representative: LyondellBasell
(86) International application number: PCT/EP2021/086926
(87) International publication number: WO 2022/136333

(56) References cited:
- WO-A1-2020/084522
- US-A- 5 608 136
- US-A1- 2012 125 064
- US-A1- 2016 040 074
- US-A1- 2019 275 486
- US-A1- 2020 103 107

## Description

### FIELD OF THE INVENTION

The present disclosure relates to the field of depolymerization of plastic waste material into new products, comprising hydrocarbon oil, which have valuable and useful properties. In one aspect, the present disclosure relates to a process for converting plastics to liquid hydrocarbons, in particular to be used as hydrocarbon feedstock.

### BACKGROUND OF THE INVENTION

The awareness that waste plastic materials have a negative impact on the environment and, as a consequence on the health of any form of life, is rapidly increasing.

One of the attempts to mitigate the impact is constituted by the recycling of plastic materials coming from domestic and industrial waste which allows a part of these materials to be reintroduced into the production cycle. This would involve further positive results such as lower use of fossil hydrocarbon sources to produce plastic items.

However, various factors indicate that this solution alone would not be sufficient for reaching the sustainability targets. In fact, mechanical recycling of plastic materials produces substances with usually lower quality, is relatively costly and burdensome and not applicable to certain urban waste in which plastic is mixed to various different materials.

As a consequence, a large part of plastic waste is either used as a source of thermal energy in plants such as incinerators, or simply stored in landfills which, as mentioned, contribute to degrade the earth environment by raising the CO₂ emissions and by the release of hazardous chemicals.

In view of the above, numerous attempts have been made in the past to efficiently reprocess a feedstock of waste plastics back into a liquid hydrocarbon product that has valuable and useful properties particularly as a fuel.

Thermocatalysis is a basic process whereby plastic waste material is converted to liquid fuel (pyrolytic product) by effect of thermal and optionally catalytical degradation in the absence of oxygen. Plastic waste is typically first melted within a stainless steel chamber under an inert purging gas, such as nitrogen. In a first thermal step this chamber heats the molten material to a gaseous state which, in a successive thermocatalytic step, is cracked to form hydrocarbon chains of variable length.

Hot pyrolytic gases are then condensed in one or more condensers to yield a hydrocarbon distillate comprising straight and branched chain aliphatic, cyclic aliphatic and aromatic hydrocarbons (pyrolytic oil). Depending on the composition, the resulting mixture is used in a variety of applications but, in any case, the requirement of product consistency and quality has always to be met. Also in view of the unstable composition of the plastic waste raw material, quality consistency of the recovered oil is difficult to be met.

The use of one or more catalytic cracking stages helps in lowering the operative degrading temperature and may also drive the product composition towards the desired target. According to US Patent 5,608,136, the amount of catalyst to be used ranges from 10 to 200% by weight relative to the amount of plastic to be treated. This greatly impacts on the costs per unit of recovered oil and, in order to be cost sustainable, it makes virtually mandatory for the plant to be endowed with a separate section for catalyst recovery.

Industrial pyrolysis or thermocatalytic plants may have two pyrolysis chambers, known as twin-chamber systems, which work in parallel at an approximately equal rate. When both chambers complete the pyrolysis of waste material in unison, it is necessary to wait for each to cool before unwanted carbonaceous char can be removed from the internal base of each chamber. In a variant, the chambers may work in alternate mode so that when one is under cleaning treatment the other is under operation. By this method however, the amount of plastic treated per unit of time is lowered.

In light of the foregoing, an object of the present disclosure is to provide for a plastic waste thermo-catalytic degradation process having, at the same time, high productivity, high throughput of liquid hydrocarbons as pyrolytic product that is of consistent quality, smooth, continuous and economic operability and possibility to being operated with a low consumption of catalyst.

### SUMMARY OF THE INVENTION

It is therefore an aspect of the present disclosure a process for depolymerizing waste plastic material and producing a pyrolytic product, wherein said process comprises the following steps:
(a) feeding a mixture comprising waste plastic materials, in an oxygen-free atmosphere, into a feeding system comprising at least one screw extruder (1), which is heated at the melting temperature of said plastic material;
(b) feeding the molten plastic material coming from the extruder into a first depolymerization reactor (2), which is a continuously stirred tank reactor maintained at a temperature ranging from 280 to 600°C and operated under a pressure ranging from 1 to 10 barg in which depolymerization takes place thereby forming a gaseous effluent and a liquid effluent;
(c) direct at least a portion of the liquid effluent produced in the first reactor (2) to a char handling section (6) and feeding the gaseous effluent from reactor (2) to a condensation unit (3) from which a gaseous stream and a liquid stream are generated;
(d) direct the gaseous stream from the first condensation unit (3) to a second condensation unit (5) working at temperature lower than the said first condensation unit and direct the liquid stream coming from the condensation unit (3) to a second depolymerization reactor (4), which is a continuously stirred tank reactor maintained at a temperature ranging from 280 to 600°C and operated under a pressure ranging from 1 to 10 barg in which depolymerization takes place thereby forming a gaseous effluent and a liquid effluent;
(e) withdrawing the gaseous effluent from said depolymerization reactor (4) and feeding it to the second condensation unit (5) and recycle at least a portion of the liquid effluent coming from depolymerization reactor (4) to the first depolymerization reactor (2);
said process being further characterized by the fact that a pyrolytic product is recovered from condensation unit (5) and that at least one of the depolymerization reactors (2) and (4) operates in the presence of a depolymerization catalyst.

### BRIEF DESCRIPTION OF DRAWINGS

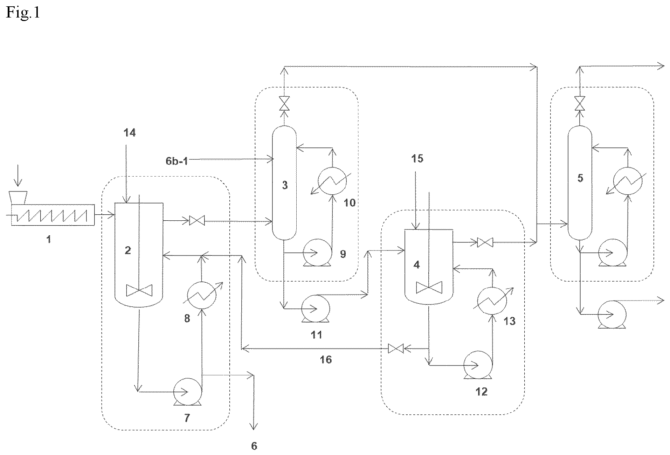

Fig. 1 is a schematic view of the thermo-catalytic process plant

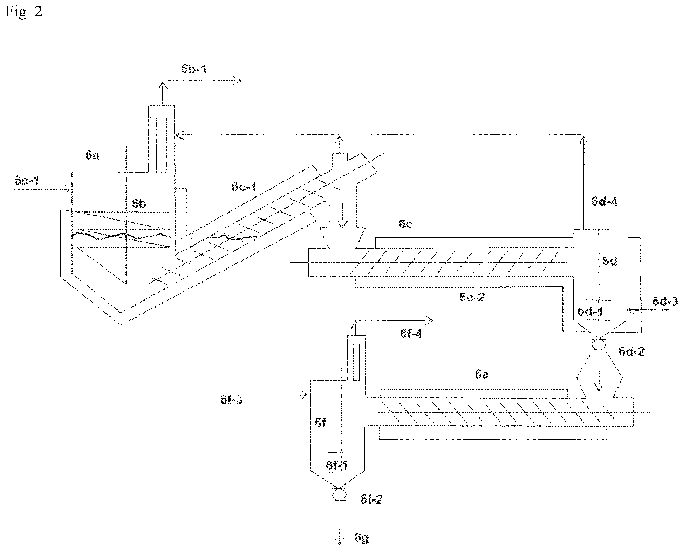

Fig. 2 is a schematic view of a first type of char handling section.
Fig. 3 is a schematic view of a second type of char handling section

### DETAILED DESCRIPTION OF THE INVENTION

Preferably, the process is carried out in a continuous mode.

In stage (a) a charging system allows charging, preferably in continuous mode, waste plastic materials to be fed, into the reactor (2). Care should be taken for not introducing oxygen containing atmosphere into the system. The barrier to the potentially oxygen-containing atmosphere can be obtained in different ways such as nitrogen blanketing or vacuum system connected to a barrel of the extruder.

More specifically, the plastic waste mixture, is charged into the feeding system of the depolymerization reactor (2) by means of a hopper, or two or more hoppers in parallel, and the oxygen present in the atmosphere of the plastic waste material is substantially eliminated inside the hopper(s).

The process according to the present disclosure is very flexible and can be fed with a wide range of plastic waste composition as, for example, a heterogeneous mixture of waste plastic materials (called Plasmix in Italy) in which polyolefins are the most abundant component but for which a further sorting step is no longer economical. It is preferred, especially when the pyrolytic product is to be recirculated back to a cracking/refining unit, to depolymerize a plastic waste mixture in which the polyolefin (PE and PP) content is equal to or higher than 70%wt.

The waste plastic material preferably undergoes a pre-treatment stage in which it is melted by heat and possibly mixed with an additive which can be an alkaline material. By the melting pretreatment, a non-uniform mixture of different kinds of waste plastics can be transformed into a mass of uniform plastic composite. Therefore, this pretreatment is also preferable for the case in which the pyrolytic decomposition is performed without additives.

The heating temperature in the pretreatment stage is appropriately set to a temperature in accordance with the kind and content of the plastic contained in the waste plastic material such that pyrolytic decomposition of the plastic material to be treated is inhibited. Such a temperature is, in general, within a range of 100°C to 300°C, and preferably, 150°C to 250°C. At a temperature close to 300°C or more, elimination of HCl from the PVC resin possibly present, takes place.

The HCl forming gas can be either removed via a venting system and successively neutralized or trapped if the waste plastic material is mixed with an alkaline material during the melting/kneading pretreatment. For performing the melting operation, ordinary kneaders, extruders with a screw and the like are applicable. Plastic waste is preferably fed to the depolymerization reactor by means of an extruder.

The extruder melts the plastic scrap, brings it at high temperature (250-350°C) and injects it into the first depolymerization reactor (2). The extruder may receive the plastic scrap cut in small pieces into the feed hopper, convey the stream in the melting section and heat the polymer by combined action of mixing energy and heat supplied by barrel heaters.

Additives can be optionally incorporated in the melt aiming at reducing corrosivity of plastic scrap received or to improve conversion process in the reaction section.

During the extrusion, one or more degassing steps can be foreseen to remove residual humidity present in the product.

Before being fed to the reactor (2), the melt stream can be filtered by in order to remove solid impurities present in the plastic waste.

Several design of melt filtration units can be applied, depending on amount and particle size of the solid impurities.

It is preferred using self-cleaning melt filters that can be operated for long time (several days) without manual intervention to replace filtration elements.

One preferred design of melt filter is based on a circular perforated plate as melt filtration element, holes by laser or by machining according to openings, where solid contaminant are accumulated. Accumulation of impurities may increase differential pressure across the melt filter. In order to perform the in line cleaning of the filtration element, a rotating scraper removes the accumulated impurities and guides them to a discharge port, that is opened for short time to purge out of the process contaminated material.

This cycle can be repeated several times (up to operation time of several days) without manual intervention or need to stop production for the time needed to replace the filtration element.

Another option of self-cleaning melt filter is based on the application of continuous filtering metal bands through which polymer flow is passed. Impurities are accumulated on the metal filter generating an increases of pressure. Accordingly the clogged filtering band section is pushed out of the polymer passage area and clean section is then inserted.

This process is automatic and allows to operate for long time (up to several days) without manual intervention or need to stop production for the time needed to replace the filtration element.

Any extrusion systems can be applied, as single screw extruders, twin screw extruders, twin screw extruders with gear pump, or combination of the above.

In step (b) the depolymerization reactor (2) is preferably an agitated vessel operated at temperature ranging from 300 to 550 and more preferably from 350 to 500°C.

The operative pressure is preferably kept in the range 2.0 to 8 barg, more preferably in the range 2.5 to 7 barg .

In order to improve mass fluidity, it constitutes a preferred embodiment premixing, preferably in a dedicated vessel, the molten mass of waste plastics entering the reactor with hydrocarbon oil, preferably recirculated oil coming from the first condensation unit, in order to promote melt dissolution into the depolymerization reactor. In this case the volumetric ratio oil/molten mass can range from 0.1:1 to 1:1.

The depolymerization reactor (2) preferably has a cylindrical section, preferably with a rounded bottom.

Preferably, it has a mixer installed in the vertical axis of the reactor, completed with a gear motor which allows the blades of the mixer rotating in order to maintain the system in stirred state. The design of the mixer and the power of the motor can vary in respect of the reactor content, volume and shape, however, as a non-limiting example, it is preferred to operate the reactor with a power input ranging from 0.2 to 4 kW/m³, preferably 0.2-2 kW/m³ and more preferably from 0.3 to 1.5 kW/m³.

The heating of the reactor takes place by means of the thermal transfer induced by a flow of molten salt, heated to a temperature ranging from 300°C to 570°C.

The feeding circuit (not shown) of the molten salt is constructed in such a way to prevent molten salt leakage. The molten salt is molten solar salt preferably constituted by a mixture of sodium nitrate and potassium nitrate, even more preferably in a weight ratio ranging from 2:3 to 3:2. The solar salt receives in turns heat from a dedicated furnace that may be either electric or be fed with fuel. In the latter case, part of the recovered oil from the condensation unit (5) may be used to feed the furnace. In the alternative, or in combination, the heat can be generated by combustion of gaseous or liquid hydrocarbons. The use of gaseous hydrocarbons is preferred.

Preferably, the heat associated to molten salt is transferred to the depolymerization reactor by circulating the molten salt through a jacket which envelops the whole reactor and/or by feed it to an external heat exchanger described below.

In both cases the salt is circulated by the use of a circulation pump. A series of fins guarantees a homogeneous distribution of the flow of molten salts in the jacket and maximization of the thermal exchange coefficient.

The depolymerization process taking place within the reactor produces molecules having reduced chain length and low boiling point. This continuously running chain breakage mechanism, particularly close to the reactor walls, produces molecules increasingly smaller part of which, at the operating temperature and pressure, are gaseous.

As a result, the composition within the reactor covers a broad range of hydrocarbons from methane to heavier products, both saturated and olefinic, with linear or highly branched structures. Some aromatic product can be also present as well as fused rings structures.

Those that are still liquid at the operating conditions, contribute to lower the liquid mass viscosity. As a result of the depolymerization process and of the composition of the feed, the content of the reactor (2) can be defined as coexistence of a liquid slurry phase, in which solid especially carbonaceous substances, and inorganic substances, are dispersed in a liquid hydrocarbon mixture, and a gaseous phase.

At least a portion of the liquid slurry phase is withdrawn from the reactor, preferably from the bottom of the reactor and constitutes the liquid effluent sent to the char handling section (6) which will be further described in detail.

From the operative point of view, the withdrawal of the slurry phase from the bottom of the reactor is preferably triggered by density sensors detecting the density of the liquid slurry reaching a predetermined value.

In a particular and preferred embodiment, part of the liquid slurry withdrawn from the the reactor (2), is recirculated, via a recycling pump (7), back to the reactor top optionally through an external heater (8). As mentioned above, heat to the external heater is preferably provided by the molten salt. This embodiment may provide both increased homogeneity of reactor content and reactor heating.

According to a preferred embodiment, the liquid slurry portion recirculated to the reactor is withdrawn from a point of the reactor different from the point of the withdrawal of the liquid slurry portion sent to the char handling,

According to another preferred embodiment, both the liquid slurry portion recirculated to the reactor and the liquid slurry portion sent to the char handling are withdrawn from the same point and then successively split.

The split between the portion of liquid slurry directed to char handling and the portion recirculated to the reactor can take place either before or after the recycling pump (7). In this latter embodiment, the liquid slurry is first fed to a dedicated vessel equipped with a lower and upper exit point. The liquid portion directed to char handling (6) is withdrawn in a concentrated form the lower exit point while the liquid portion to be recycled to the reactor (2) is withdrawn from the upper exit point.

The gaseous phase of the reactor (2) constitutes the gaseous effluent which is sent to the condensation unit (3) for further treatment.

The gaseous effluent comprises a mixture of light hydrocarbons which may also include some heavy hydrocarbons and char particles entrained. The gaseous effluent are conveyed from the reactor top to a condenser (3) preferably operated at a pressure slightly lower than that of the reactor .

The condenser (3) is better designed as a scrubber column in order to suppress the entrained char. The condenser temperature is selected in such a way that the heavy hydrocarbons are condensed and the light hydrocarbons are released as gaseous stream. The gaseous stream (H₂ and light hydrocarbons) is conveyed to the further condensation unit (5) working at a temperature lower than the condensation unit (3) from which oil is recovered.

The operative temperature of the condensing unit (3) may vary in a wide range also depending on the operative pressure. The temperature, referred to atmospheric pressure, can be from 20°C to 200°C more preferably from 50 to 200°C and especially from 60 to 180°C. The temperature range can be of course different when a higher operating pressure is chosen.

In a particular embodiment, the condenser unit (3) was operated at about 80°C and the condensed liquid was sampled and analyzed with GC analysis.

Due to the very high number of compounds, the result of the analysis has been reported by grouping the resulting compounds according to their retention time using specific hydrocarbons as internal retention time references. Results may show the presence of about 2wt% or more of compounds with retention time equal to, or less than, n-heptane, about 25wt% or more of compounds with retention time comprised between n-heptane and n-dodecane, a more abundant fraction of compounds having a retention time higher than n-dodecane and lower than n-octacosane (70wt% or less) and a possible presence, in a small amount, of a fraction with higher retention time.

In a preferred setup of the condensation unit (3) a dephlegmator (partial condenser) is installed on top of the scrubber and works at a temperature lower than that inside the column. The condensate flows down as reflux for the scrubber by virtue of gravity. The dephlegmator can either be installed as a separate piece of equipment or inside the scrubber.

In an alternative preferred setup, a pump recycles the liquid that collects in the bottom of the scrubber to the top of the column. The recycled liquid is cooled in a dedicated heat exchanger before injection into the scrubber top as reflux.

The hydrocarbon condensate, preferably having more than C7 carbon atoms, constitutes the liquid stream which is transferred by means of a pump to the second depolymerization reactor (4).

The second depolymerization reactor (4) is preferably of the same type as the first one and more preferably a continuously stirred tank reactor.

Depolymerization takes place in the same range of temperatures but, in order to limit the volatility of the heavy hydrocarbons, it is preferably operated at a pressure higher than the first reactor and in particular in the range from 2 to 10 barg, preferably from 3 to 9 barg and more preferably from 3 to 8 barg.

Since the reactor (4) is fed with the condensed effluents coming from the reactor (2) it contains less impurities and produces less char. Preferably, fresh catalyst is fed to reactor (4) through conduit (15).

According to the present disclosure, the catalyst can be selected from those active as depolymerization/cracking catalysts in thermocatalytic processes. In particular, it can be selected from metal oxides, heteropolyacids, mesoporous silica, aluminosilicates catalysts, such as halloysite and kaolinite, and preferably from zeolites. Among them, particularly preferred zeolites are synthetic Y-type zeolite and ZSM-5.

In a particularly preferred embodiment, the amount of catalyst feed is not more than 10% preferably not more than 5% and especially not more than 2% wt with respect to the plastic waste feed.

In a preferred embodiment, the catalyst is injected into the second reactor as powder dispersed into a hydrocarbon oil preferably the liquid pyrolytic product (oil) obtained from condensation units (3) or (5), preferably from condensation unit (3).

Preferably, the catalyst slurry is prepared in a pot, continuously stirred vessel where the catalyst is poured from a dedicated silo in order to keep constant the concentration of the catalyst in the slurry.

The pyrolytic oil dispersing the catalyst is preferably withdrawn from the condensation unit (3) in order to keep constant the slurry level in the pot. Once ready the catalyst slurry can be injected, preferably into the second reactor, preferably by means of a progressive cavity pump in order to keep its level constant.

The liquid effluent coming from reactor (4) is preferably a highly concentrated hydrocarbon slurry which preferably contains the depolymerization catalyst. It is discharged from the second reactor and sent back to the first reactor via the conduit (16). The same density control in reactor (4) for the withdrawal of the slurry is preferably operated also for reactor (2).

The slurry density can be control via available methods such as γ-ray measurement or Coriolis densimeter. When the operative pressure of the first reactor is lower than the second one, the light hydrocarbons of the slurry entering the first reactor are expected to vaporize and be extracted with the gaseous effluent produced in reactor (2).

Preferably, the amount of slurry recycled to the first reactor ranges from 5 to 40% in volume more preferably from 10 to 30% in volume of the second reactor content.

Also in reactor (4) it constitutes a preferred embodiment that part of the liquid slurry withdrawn from the bottom of the reactor (4) is recirculated, with a recycling pump (12), back to the reactor top through an external heater (13).

The gaseous effluent produced from reactor (4) is conveyed to the condensation unit (5) for the recovering of the pyrolytic product in form of an oil.

The condensation unit (5) has preferably a similar configuration to condensation unit (3).

Preferably, the operative conditions of the condensation unit (5) are selected in a way that it has a lower operating temperature and pressure with respect to condensation unit (3).

In particular, the temperature may range from 20 to 80°C and preferably from 30 to 70°C. The pressure value should preferably be lower than that of condensation unit (3) so as to allow incondensable gases from unit (3) to enter unit (5) without further pressurization. The oil recovered from the condensation unit 5 is generally lighter than that recovered from the first condensation unit and may in particular have following composition (GC determined):
- about 10-15% wt% of a fraction having retention time equal or less then n-heptane;
- about 70-75wt% of a fractions with retention time comprised by n-heptane and n-dodecane, and
- about 12-20 wt% of product having a retention time higher than n-dodecane and lower than n-octacosane,
- no traces of compounds with higher retention time

As mentioned above, the liquid effluent discharged from the pyrolysis reactor (2) and directed to the char handling section is in the form of slurry, and especially highly concentrated slurry and is preferably discharged continuously. Operating a pressurized reactor makes possible to easily discharge a concentrated slurry to a lower pressure device without using additional withdrawal equipment. As mentioned, the slurry may be discharged by the bottom of the reactor, or, when present, from the line or vessel after the recycling pump (7).

In view of process set-up the flow of the slurry stream is preferably continuous. The char content in the slurry may range from 10 to 50% preferably from 20 to 40%wt.

One preferred char handling section is characterized by comprising
- a first jacketed chamber (6a), provided with inlet conduit (6a-1) for feeding a char containing slurry and stirring system (6b), capable to be operated at a temperature ranging from 350 to 570°C and further provided with a conduit (6b-1) for the withdrawal of a gaseous effluent and
- means (6c) for withdrawing a concentrated slurry from chamber (6a) and conveying it to a second chamber (6d) said means (6c) being capable to maintain the slurry in the same temperature range of chamber (6a) and being provided with a degassing system for removal of a gaseous effluent and producing a drained char;
- a second stripping chamber (6d), receiving the drained char, equipped with stirring system (6d-1), a char outlet (6d-2) a gas inlet (6d-3) located in the bottom of said chamber, and with a gas outlet (6d-4) for removal of gaseous effluent stripped out from the char;
- means (6e) for receiving the dry char of stripping chamber (6d) and conveying it to a third collecting chamber (6f) said means (6e) being capable to maintain the char at a temperature ranging from 60 to 100°C;
- a third collecting chamber (6f) receiving the dry char provided with stirring system (6f-1), an outlet for char disposal operated by a valve (6f-2).

The char handling process has the purpose of isolating and disposing the solid portion of the plastic feed that cannot anymore depolymerize.

In a preferred embodiment, the hot slurry withdrawn from reactor (2) is sent to the jacketed chamber (6a) operating at a temperature ranging from 350 to 570°C in which, by effect of the heat transferred by the hot jacket, depolymerization process can take place under stirring provided by the rotating blades (6b). The gaseous effluent produced, exits the chamber (6a) and is preferably sent to the condensation unit (3) and treated together with the gaseous effluent of the first reactor (2). Preferably, a filtering means is installed on the gas outlet conduit with the purpose of blocking entrained particles of char. In a particular embodiment, the gaseous effluent is first condensed in a dedicated unit and then sent back, by a pumping unit, to the condensation unit (3).

A concentrated slurry (mud) is formed and withdrawn from the bottom of chamber (6a) by a screw (hot) conveyor (6c) which transfers it to a the chamber (6d). The screw conveyor (6c) is preferably jacketed so as it can work at the same temperature range of chamber (6a). In a more still preferred embodiment, molten solar salt is circulated within the jackets of chamber (6a) and screw conveyor (6c).

The screw conveyor (6c) is preferably made of two distinct conveyors connected to each other. Preferably, the concentrated slurry, which is preferably in the form of a mud, is withdrawn from the bottom chamber (6a) by a first lifting screw conveyor (6c-1) having its lower end integrated with the bottom of chamber (6a) and its upper end, positioned at a higher height with respect to the lower end, connected to one end of a second screw conveyor (6c-2) having the other end connected and feeding the upper portion of the stripping chamber (6d). Both conveyors are jacketed and work in the same temperature range of chamber (6a).

While being elevated from the bottom of chamber (6a) and transferred via the second conveyor to stripping chamber (6d) the mud becomes progressively drained due to partial flowing back of liquid into chamber (6a) and partial conversion into gaseous effluent that is removed.

The above mentioned system allows the stripping chamber (6d) being fed with a char material containing a limited amount of liquid and preferably being partially dried.

In the stripping chamber (6d), nitrogen is preferably injected into the bottom of the stripping chamber in order to remove volatile hydrocarbon products. From the top of the stripping chamber (6d) the gases are withdrawn and sent to the condensation unit (3) while from the bottom of the chamber the dry char is discharged, preferably via a ball valve, into a further jacketed screw conveyor (6e) maintained at a temperature ranging from 60 to 100°C which conveys the char directly into a stirred collecting chamber (6f) which preferably operates at about atmospheric pressure and a temperature ranging from 60 and 100°C. More preferably, in the collecting chamber a nitrogen circulation is maintained via a gas inlet (6f-3) and a gas outlet (6f-4). The finally dry char is discharged via the valve (6f-2) preferably into a mobile container (6g) and then disposed.

The above disclosed process and apparatus for char dislodging handling and disposal is very flexible and it allows to be operated in a continuous mode without the need to stop depolymerization in reactor (2). This makes also possible to have a lighter depolymerization plant set up because it is no longer strictly required to have reactors working in parallel in order to run a continuously working depolymerization process.

The chair handling process is also very efficient releasing a dried char with typical composition of about 20% wt exhausted catalyst, 50%wt inorganics and about 30%wt carbon.

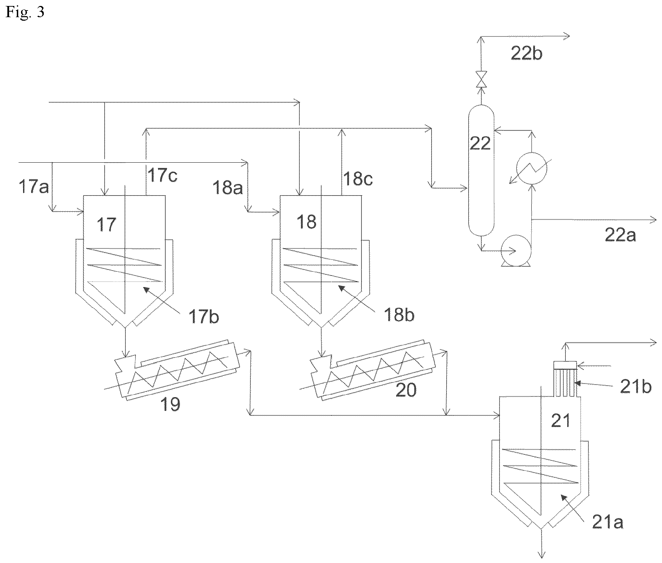

In another embodiment, according to Fig. 3, the char handling section may comprise two or more jacketed chambers (17, 18), provided with inlet conduit (17a, 18a) for feeding the char containing slurry and stirring system (17b, 18b), capable to be operated at a temperature ranging from 350 to 570°C and further provided with a conduit (17c; 18c) for the withdrawal of a gaseous effluent and with a conduit (17d, 18d) for withdrawing a dried char to be sent to a conveyor mean;

means (19, 20) for conveying the char to a collecting chamber (21) said means (19, 20) being capable to lower the temperature of the char exiting the couple of jacketed chambers (17, 18);

a collecting chamber (21) receiving the dry char provided with stirring system (21a), an outlet for char disposal.

The discharged slurry is preferably conveyed in two twin devolatilization chambers.

Each devolatilization chambers preferably work in alternating and opposite modes. In the first mode (accumulation), the chamber receives and collects the discharged slurry. In the second mode (drying) the chamber provides residence time to a thermal treatment during which the heavy oil is either vaporized or further pyrolyzed in light oil.

Preferably, when one devolatilization chamber works in the accumulation mode, the other works in the drying mode. Preferably, they are synchronized so that they complete one mode at, substantially, the same time and can swap to the other mode. This ensures a constant output of dried char along the time.

At the end of the thermal treatment, the char is preferably substantially dried.

Pyrolytic gases relieved by devolatilization chambers are conveyed to a dedicated condensation section (22), which operates in analogy with condensation section (3).

Preferably, pyrolytic vapors are suppressed and condensed at 80-100°C. The condensation temperature is selected in order to avoid the formation of wax. The condensate is preferably recycled via conduit (22a) to the depolymerization reactor (2).

The non-condensable fraction consists mainly of nitrogen entraining few hydrocarbons and is considered as waste gas exiting via conduit 22b.

The devolatilization chambers are agitated and jacketed vessels. Preferably, the heat transfer fluid is solar salt, by means of which high operating temperatures may be reached (500-550°C).

The removal of the pyrolytic vapors from devolatilization chambers is preferably enhanced by flushing of stripping nitrogen.

Preferably, devolatilization chambers and dedicated condensation section are connected and operated at a pressure ranging from 1 to 3.0 barg.

When char is fully dried it is discharged into a jacketed screw conveyor (19, 20) especially designed for cooling the char.

The screw conveyors cool down the dried char. Char sensible heat is preferably removed by means of cold water flowing through one or more of body jacket, screw shaft and screw flight (hollow flight).

The dried and cold char is collected in a prechamber (21) before the discharge for disposal.

The prechamber is endowed with jacket (cold water), agitator (21a) and bag filter (21b) which prevents the entrainment of powder. The filtered nitrogen is considered as waste gas.
The so designed depolymerization process is very efficient because it can produce about 10%wt of pyrolytic gas, about 80% wt pyrolytic oil and about 10% wt of char.
As already mentioned, the preferred use of the main product of the pyrolytic process of the disclosure is as hydrocarbon feedstock partially replacing oil feedstock in cracking plants. However, other uses, such as fuel, are also contemplated.

### EXAMPLES

### Example 1

The following experimental steps have been carried out in a depolymerization apparatus two reactors connected in series , consisting of a mechanically agitated vessel (and jacketed for heating). The first reactor was provided with an inlet for the plastic waste coming from the extruder feed, and an outlet for the generated gases. The gases withdrawn from the reactor are conveyed to a condensation unit from which an incondensable gas and a pyrolytic oil are obtained. Thermocouples are positioned into the reactor to monitor and record the temperatures. The oil collected from the condensation unit is fed to a second depolymerization reactor, provided also with an inlet for catalyst feeding The catalyst was fed into the reactor as solid slurry by mixing with part of the same oil from the condensation section.

The second reactor was also provided with an outlet line in order to recycle part of the reactor content to the first depolymerization reactor.

The plastic waste feedstock was previously analyzed to check the polyolefin content (97wt%) with the residual containing traces of other common polymers (PET, PS, PA, PU) plus inorganic contaminants.

The feedstock was homogeneized and pelletized before the loading in the hopper needed to feed the extruder which worked at a temperature of 290°C and discharged continuously into the depolymerization reactor at 4 kg/h. The first depolymerization reactor was operated at a pressure of 3 barg and at temperature of about 408°C while the average residence time was about 3h. The gaseous phase of the reactor was sent to a condensation unit formed by a cooling/scrubber column working at 80°C and a dephlegmator working at 25°C. Then, the oil stream was fed into the second vessel operated at 398°C and 5 barg. Average residence time in this case was about 105 minutes. In this second reactor, a sample of H-USY Zeolite type (CBV 400 - CAS number 1318-02-1 ex Zeolyst International) was tested. The catalyst was fed into the pyrolizer in such an amount to get a ratio of 6wt% with respect to the reactive phase mass.

As mentioned above, about 10% volume of the reactor content was recycled to the first reactor while the gas outlet from the second reactor was sent to a second condenser also kept at 25°C, where the pyrolitic oil is condensed. The not condensable gaseous stream was conveyed to vent.

The condensed oil was analyzed via GC-FID.

Due to the very high number of compounds, the result of the analysis has been reported by grouping the resulting compounds according to their retention time using specific hydrocarbons as internal retention time references. Results are reported in Table 1.

### Example 2

The process set up reported in Ex.1 was duplicated but the reactor pressure was kept at 6barg. Average residence time in the second reactor was about 150 minutes. Results are reported in Table 1.

### Comparative Example 3

A similar test was performed as described in Ex.1 but the stream of gases from the first reactor, was directly sent to the final condensation unit operated at the same manner. Moreover, nAs it results from Table 1, the quality of the oil composition worsened.

### Comparative Example 4.

The run was carried out according to the conditions reported in Example 1 with the difference that the content of the second reactor was not recycled to the first one. In order to keep the pyrolytic oil quality at the same level, the residence time in the first reactor was 3.2 hours and the feedstock feeding reduced to 3.3 kg/h.

**Table 1**

| Ex | Cat. | BP<98°C | 98°C<BP<203°C | 203°C<BP<365°C | 365°C<BP<434°C | BP>434°C |
|---|---|---|---|---|---|---|
| Ex. 1 | H-Y | 1 | 44 | 55 | 0 | 0 |
| Ex.2 | H-Y | 2 | 44 | 54 | 0 | 0 |
| C.3 | no | 2 | 40 | 53 | 3 | 2 |
| C. 4 | H-Y | 2 | 44 | 55 | 0 | 0 |

## Claims

1. A process for depolymerizing waste plastic material and producing a pyrolytic product, wherein said process comprises the following steps:
(a) feeding a mixture comprising waste plastic materials, in an oxygen-free atmosphere, into a feeding system comprising at least one screw extruder (1), which is heated at melting temperature of said plastic material;
(b) feeding the molten plastic material coming from the extruder into a first depolymerization reactor (2), which is a continuously stirred tank reactor maintained at a temperature ranging from 280 to 600°C and operated under a pressure ranging from 1 to 10 barg in which depolymerization takes place thereby forming a gaseous effluent and a liquid effluent;
(c) direct at least a portion of the liquid effluent produced in the first reactor (2) to a char handling section (6) and feeding the gaseous effluent from reactor (2) to a condensation unit (3) from which a gaseous stream and a liquid stream are generated;
(d) direct the gaseous stream from the first condensation unit (3) to a second condensation unit (5) working at temperature lower than the said first condensation unit and direct the liquid stream coming from the condensation unit (3) to a second depolymerization reactor (4), which is a continuously stirred tank reactor maintained at a temperature ranging from 280 to 600°C and operated under a pressure ranging from 1 to 10 barg in which depolymerization takes place thereby forming a gaseous effluent and a liquid effluent;
(e) withdrawing the gaseous effluent from said depolymerization reactor (4) and feeding it to the second condensation unit (5) and recycle at least a portion of the liquid effluent coming from depolymerization reactor (4) to the first depolymerization reactor (2);
said process being further **characterized by** the fact that a pyrolytic product is recovered from condensation unit (5) and that at least one of the depolymerization reactors (2) and (4) operates in the presence of a depolymerization catalyst.

2. The process according to any of the preceding claims in which plastic waste is a mixture of waste materials in which polyolefins are the most abundant component.

3. The process according to any of the preceding claims in which the depolymerization reactor (2) is preferably an agitated vessel operated at temperature ranging from 300 to 550°Cand more preferably from 350 to 500°C. and under a pressure kept in the range 2.0 to 8 barg, more preferably in the range 2.5 to 7 barg.

4. The process according to any of the preceding claims in which the molten mass of waste plastics entering the reactor is premixed with hydrocarbon oil.

5. The process according to any of the preceding claims in which the heating of the reactor takes place by means of the thermal transfer induced by a flow of molten salt, heated to a temperature ranging from 300°C to 570°C and circulated within reactor jacket.

6. The process according to any of the preceding claims in which part of the liquid slurry withdrawn from the bottom of the reactor (2) is recirculated, via a recycling pump (7), back to the reactor top.

7. The process according to any of the preceding claims in which the condenser (3) operates at a pressure lower than that of the reactor (2).

8. The process according to any of the preceding claims in which the condenser (3) is a scrubber column in which heavy hydrocarbons are condensed and the light hydrocarbons are released as gaseous stream.

9. The process according to any of the preceding claims in which the second depolymerization reactor (4) is operated at a temperature ranging from 280 to 600°C and at a pressure higher than the first reactor and in particular in the range from 2 to 10 barg.

10. The process according to any of the preceding claims in which a fresh depolymerization catalyst is fed to reactor (4).

11. The process according to any of the preceding claims in which the pyrolytic product in form of oil recovered from the condensation unit (5) has the following composition (GC determined):
- about 10-15% wt% of a fraction having retention time equal or less then n-heptane;
- about 70-75wt% of a fractions with retention time comprised by n-heptane and n-dodecane, and
about 12-20 wt% of product having a retention time higher than that of n-dodecane and lower than that of n-octacosane.

12. The process according to any of the preceding claims in which the pyrolytic product in form of oil recovered from the second condensation unit is used as hydrocarbon feedstock in cracking plants.

13. The process according to any of the preceding claims in which at least a portion of the liquid effluent produced in the first reactor (2) is directed to a char handling section comprising:
- a first jacketed chamber (6a), provided with inlet conduit (6a-1) for feeding char containing slurry and stirring system (6b), capable to be operated at a temperature ranging from 350 to 570°C and further provided with a conduit (6b-1) for the withdrawal of a gaseous effluent and
- means (6c) for withdrawing a concentrated slurry from chamber (6a) and conveying it to a second chamber (6d) said means (6c) being capable to maintain the slurry in the same temperature range of chamber (6a) and being provided with a degassing system for removal of a gaseous effluent and producing a drained char;
- a second stripping chamber (6d), receiving the drained char, equipped with stirring system (6d-1), a char outlet (6d-2) a gas inlet (6d-3) located in the bottom of said chamber, and with a gas outlet (6d-4) for removal of gaseous effluent stripped out from the char;
- means (6e) for receiving the dry char of stripping chamber (6d) and conveying it to a third collecting chamber (6f) said means (6e) being capable to maintain the char at a temperature ranging from 60 to 100°C;
- a third collecting chamber (6f) receiving the dry char provided with stirring system (6f-1), an outlet for char disposal operated by a valve (6f-2).

14. The process according to any of the claims 1-12 in which at least a portion of the liquid effluent produced in the first reactor (2) is directed to a char handling section comprising
- two or more jacketed chambers (17, 18), provided with inlet conduit (17a, 18a) for feeding the char containing slurry and stirring system (17b, 18b), capable to be operated at a temperature ranging from 350 to 570°C and further provided with a conduit (17c; 18c) for the withdrawal of a gaseous effluent and with a conduit (17d, 18d) for withdrawing a dried char to be sent to conveyor means;
- means (19, 20) for conveying the char to a collecting chamber (21) said means (19, 20) being capable to lower the temperature of the char exiting the couple of jacketed chambers (17, 18); and
- a collecting chamber (21) receiving the dry char provided with stirring system (21a), and an outlet for char disposal.

15. The process according to any of the preceding claims in which the char content in the liquid effluent effluent produced in the first reactor (2) in the form of a slurry and directed to the char handling section ranges from 10 to 50% preferably from 20 to 40%wt based on the total weight of the slurry.

## Patentansprüche

1. Verfahren zur Depolymerisation von Kunststoffabfallmaterial und zur Produktion eines pyrolytischen Produkts, wobei das Verfahren die folgenden Schritte umfasst:
(a) Einspeisen einer Mischung, die Kunststoffabfallmaterialien umfasst, in einer sauerstofffreien Atmosphäre in ein Einspeisesystem, das mindestens einen Schneckenextruder (1) umfasst, der auf Schmelztemperatur des Kunststoffmaterials erhitzt ist;
(b) Einspeisen des geschmolzenen Kunststoffmaterials, das aus dem Extruder kommt, in einen ersten Depolymerisationsreaktor (2), der ein kontinuierlich gerührter Tankreaktor ist, der auf einer Temperatur im Bereich von 280 bis 600 °C gehalten wird und unter einem Druck im Bereich von 1 bis 10 bar Manometerdruck (barg) betrieben wird, in dem die Depolymerisation stattfindet, wodurch ein gasförmiger Ausfluss und ein flüssiger Ausfluss gebildet werden;
(c) Lenken von mindestens einem Anteil des in dem ersten Reaktor (2) produzierten flüssigen Ausflusses in einen Kohlebehandlungsabschnitt (6) und Einspeisen des gasförmigen Ausflusses aus Reaktor (2) in eine Kondensationseinheit (3), aus der ein gasförmiger Strom und ein flüssiger Strom erzeugt werden;
(d) Lenken des gasförmigen Stroms aus der ersten Kondensationseinheit (3) in eine zweite Kondensationseinheit (5), die bei einer Temperatur arbeitet, die niedriger als diejenige der ersten Kondensationseinheit ist, und Lenken des von der Kondensationseinheit (3) kommenden flüssigen Stroms in einen zweiten Depolymerisationsreaktor (4), der ein kontinuierlich gerührter Tankreaktor ist, der auf einer Temperatur im Bereich von 280 bis 600 °C gehalten und unter einem Druck im Bereich von 1 bis 10 barg betrieben wird, in dem Depolymerisation stattfindet, wodurch ein gasförmiger Ausfluss und ein flüssiger Ausfluss gebildet werden;
(e) Abziehen des gasförmigen Ausflusses aus dem Depolymerisationsreaktor (4) und Einspeisen desselben in die zweite Kondensationseinheit (5), und Recyceln von mindestens einem Anteil des flüssigen Ausflusses, der aus dem Depolymerisationsreaktor (4) kommt, in den ersten Depolymerisationsreaktor (2);
wobei das Verfahren des Weiteren durch die Tatsache gekennzeichnet ist, dass aus Kondensationseinheit (5) ein pyrolytisches Produkt gewonnen wird, und dass mindestens einer der Depolymerisationsreaktoren (2) und (4) in Gegenwart eines Depolymerisationskatalysators betrieben wird.

2. Verfahren nach einem der vorhergehenden Ansprüche, wobei der Kunststoffabfall eine Mischung aus Abfallmaterialien ist, in der Polyolefine die häufigste Komponente sind.

3. Verfahren nach einem der vorhergehenden Ansprüche, wobei der Depolymerisationsreaktor (2) vorzugsweise ein bewegtes Gefäß ist, das bei einer Temperatur im Bereich von 300 bis 550 °C und bevorzugter 350 bis 500 °C und unter einem Druck betrieben wird, der im Bereich von 2,0 bis 8 barg, bevorzugter im Bereich von 2,5 bis 7 barg gehalten wird.

4. Verfahren nach einem der vorhergehenden Ansprüche, wobei die geschmolzene Masse der Kunststoffabfälle, die in den Reaktor eintritt, mit Kohlenwasserstofföl vorgemischt wird.

5. Verfahren nach einem der vorhergehenden Ansprüche, wobei das Erhitzen des Reaktors mittels Wärmeübertragung stattfindet, die durch einen Fluss aus geschmolzenem Salz induziert wird, der auf eine Temperatur im Bereich von 300 °C bis 570 °C erhitzt und in der Reaktorummantelung zirkuliert wird.

6. Verfahren nach einem der vorhergehenden Ansprüche, wobei ein Teil der aus dem Sumpf des Reaktors (2) abgezogenen flüssigen Aufschlämmung mittels einer Recyclingpumpe (7) zurück in den oberen Bereich des Reaktors rezirkuliert wird.

7. Verfahren nach einem der vorhergehenden Ansprüche, wobei der Kondensator (3) mit einem Druck betrieben wird, der niedriger als derjenige des Reaktors (2) ist.

8. Verfahren nach einem der vorhergehenden Ansprüche, wobei der Kondensator (3) eine Waschkolonne ist, in der schwere Kohlenwasserstoffe kondensiert werden und die leichten Kohlenwasserstoffe als gasförmiger Strom freigesetzt werden.

9. Verfahren nach einem der vorhergehenden Ansprüche, wobei der zweite Depolymerisationsreaktor (4) bei einer Temperatur im Bereich von 280 bis 600 °C und einem Druck betrieben wird, der höher als derjenige in dem ersten Reaktor ist und insbesondere im Bereich von 2 bis 10 barg liegt.

10. Verfahren nach einem der vorhergehenden Ansprüche, wobei in den Reaktor (4) frischer Depolymerisationskatalysator eingespeist wird.

11. Verfahren nach einem der vorhergehenden Ansprüche, wobei das pyrolytische Produkt in Form von Öl, das aus der Kondensationseinheit (5) gewonnen wird, die folgende Zusammensetzung aufweist (bestimmt mittels GC):
- etwa 10 bis 15 Gew.% einer Fraktion mit einer Retentionszeit gleich oder kleiner als n-Heptan;
- etwa 70 bis 75 Gew.% von Fraktionen mit einer Retentionszeit, die von n-Heptan und n-Dodecan umfasst wird, und
- etwa 12 bis 20 Gew.% von Produkt mit einer Retentionszeit, die höher als diejenige von n-Dodecan und niedriger als diejenige von n-Octacosan ist.

12. Verfahren nach einem der vorhergehenden Ansprüche, wobei das pyrolytische Produkt in Form von Öl, das aus der zweiten Kondensationseinheit gewonnen wird, als Kohlenwasserstoffeinsatzmaterial in Crack-Anlagen verwendet wird.

13. Verfahren nach einem der vorhergehenden Ansprüche, wobei mindestens ein Anteil des in dem ersten Reaktor (2) produzierten flüssigen Ausflusses zu einem Kohlebehandlungsabschnitt gelenkt wird, umfassend:
- eine erste ummantelte Kammer (6a), die mit einer Einlassleitung (6a-1), um kohlehaltige Aufschlämmung einzuspeisen, und einem Rührsystem (6b) ausgestattet ist, das bei einer Temperatur im Bereich von 350 bis 570 °C betrieben werden kann, und des Weiteren mit einer Rohrleitung (6b-1) zum Abziehen eines gasförmigen Ausflusses ausgestattet ist, und
- Mittel (6c) zum Abziehen einer konzentrierten Aufschlämmung aus Kammer (6a) und Transportieren derselben zu einer zweiten Kammer (6d), wobei das Mittel (6c) in der Lage ist, die Aufschlämmung in demselben Temperaturbereich wie Kammer (6a) zu halten, und mit einem Entgasungssystem ausgestattet ist, um einen gasförmigen Ausfluss zu entfernen und eine abgelassene Kohle zu produzieren;
- eine zweite Strippkammer (6d), die die abgelassene Kohle aufnimmt, mit einem Rührsystem (6d-1), einem Kohleauslass (6d-2), einem Gaseinlass (6d-3), der sich im Sumpf der Kammer befindet, und mit einem Gasauslass (6d-4) ausgestattet ist, um gasförmigen Ausfluss zu entfernen, der aus der Kohle gestrippt wurde;
- Mittel (6e) zum Aufnehmen der trockenen Kohle der Strippkammer (6d) und Transportieren derselben zu einer dritten Sammelkammer (6f), wobei das Mittel (6e) in der Lage ist, die Kohle auf einer Temperatur im Bereich von 60 bis 100 °C zu halten;
- eine dritte Sammelkammer (6f), die die trockene Kohle aufnimmt und mit einem Rührsystem (6f-1) und einem Auslass zur Kohlebeseitigung ausgestattet ist, der mit einem Ventil (6f-2) betrieben wird.

14. Verfahren nach einem der Ansprüche 1 bis 12, wobei mindestens ein Anteil des in dem ersten Reaktor (2) produzierten flüssigen Ausflusses zu einem Kohlebehandlungsabschnitt gelenkt wird, umfassend:
- zwei oder mehr ummantelte Kammern (17, 18), die mit Einlassleitungen (17a, 18a), um die kohlehaltige Aufschlämmung einzuspeisen, und einem Rührsystem (17b, 18b) ausgestattet sind, das bei einer Temperatur im Bereich von 350 bis 570 °C betrieben werden kann, und des Weiteren mit Rohrleitungen (17c; 18c) zum Abziehen eines gasförmigen Ausflusses und mit Rohrleitungen (17d, 18d) zum Abziehen von getrockneter Kohle ausgestattet ist, die zu Transportmitteln gesendet werden soll;
- Mitteln (19, 20) zum Transportieren der Kohle zu einer Sammelkammer (21), wobei die Mittel (19, 20) in der Lage sind, die Temperatur der aus der Mehrzahl von ummantelten Kammern (17, 18) austretenden Kohle abzusenken; und
- eine Sammelkammer (21), die die trockene Kohle aufnimmt und mit einem Rührsystem (21a) und einem Auslass zur Kohlebeseitigung ausgestattet ist.

15. Verfahren nach einem der vorhergehenden Ansprüche, wobei der Kohlegehalt in dem flüssigen Ausfluss, der in dem ersten Reaktor (2) in Form einer Aufschlämmung produziert wird und zu dem Kohlebehandlungsabschnitt gelenkt wird, im Bereich von 10 bis 50 Gew.%, vorzugsweise 20 bis 40 Gew.% liegt, bezogen auf das Gesamtgewicht der Aufschlämmung.

## Revendications

1. Procédé de dépolymérisation de déchets plastiques et de production d'un produit pyrolytique, ledit procédé comprenant les étapes suivantes :
(a) introduction d'un mélange comprenant des déchets plastiques, dans une atmosphère exempte d'oxygène, dans un système d'introduction comprenant au moins une extrudeuse à vis (1), qui est chauffée à la température de fusion du matériau plastique ;
(b) introduction de la matière plastique fondue provenant de l'extrudeuse dans un premier réacteur de dépolymérisation (2), qui est un réacteur à cuve agitée en continu maintenu à une température située dans la plage de 280 à 600 °C et fonctionnant sous une pression située dans la plage de 1 à 10 barg, dans lequel a lieu la dépolymérisation, formant ainsi un effluent gazeux et un effluent liquide ;
(c) guidage d'au moins une partie de l'effluent liquide produit dans le premier réacteur (2) vers une section de manipulation de produit de carbonisation (6) et introduction de l'effluent gazeux depuis le réacteur (2) dans une unité de condensation (3) à partir de laquelle un flux gazeux et un flux liquide sont générés ;
(d) guidage du flux gazeux depuis la première unité de condensation (3) vers une deuxième unité de condensation (5) travaillant à une température inférieure à celle de ladite première unité de condensation et guidage du flux liquide provenant de l'unité de condensation (3) vers un deuxième réacteur de dépolymérisation (4), qui est un réacteur à cuve agitée en continu maintenu à une température située dans la plage de 280 à 600 °C et fonctionnant sous une pression située dans la plage de 1 à 10 barg, dans lequel a lieu la dépolymérisation, formant ainsi un effluent gazeux et un effluent liquide ;
(e) soutirage de l'effluent gazeux à partir dudit réacteur de dépolymérisation (4) et son introduction dans la deuxième unité de condensation (5) et recyclage d'au moins une partie de l'effluent liquide provenant du réacteur de dépolymérisation (4) vers le premier réacteur de dépolymérisation (2) ;
ledit procédé étant en outre **caractérisé en ce qu'**un produit pyrolytique est récupéré à partir de l'unité de condensation (5) et qu'au moins l'un des réacteurs de dépolymérisation (2) et (4) fonctionne en présence d'un catalyseur de dépolymérisation.

2. Procédé selon l'une quelconque des revendications précédentes, dans lequel les déchets plastiques sont un mélange de déchets dans lesquels les polyoléfines sont le composant le plus abondant.

3. Procédé selon l'une quelconque des revendications précédentes, dans lequel le réacteur de dépolymérisation (2) est de préférence un récipient agité fonctionnant à une température située dans la plage de 300 à 550 °C et plus préférablement de 350 à 500 °C et sous une pression maintenue dans la plage de 2,0 à 8 barg, plus préférablement dans la plage de 2,5 à 7 barg.

4. Procédé selon l'une quelconque des revendications précédentes, dans lequel la masse fondue de déchets plastiques entrant dans le réacteur est prémélangée avec une huile hydrocarbonée.

5. Procédé selon l'une quelconque des revendications précédentes, dans lequel le chauffage du réacteur a lieu au moyen du transfert thermique induit par un écoulement de sel fondu, chauffé à une température située dans la plage de 300 °C à 570 °C et mis en circulation à l'intérieur de la chemise du réacteur.

6. Procédé selon l'une quelconque des revendications précédentes, dans lequel une partie de la suspension liquide soutirée à partir du fond du réacteur (2) est remise en circulation, via une pompe de recyclage (7), en retour vers le haut du réacteur.

7. Procédé selon l'une quelconque des revendications précédentes, dans lequel le condenseur (3) fonctionne à une pression inférieure à celle du réacteur (2).

8. Procédé selon l'une quelconque des revendications précédentes, dans lequel le condenseur (3) est une colonne d'épuration dans laquelle des hydrocarbures lourds sont condensés et les hydrocarbures légers sont libérés sous forme de flux gazeux.

9. Procédé selon l'une quelconque des revendications précédentes, dans lequel le deuxième réacteur de dépolymérisation (4) fonctionne à une température située dans la plage de 280 à 600 °C et à une pression supérieure à celle du premier réacteur et en particulier située dans la plage de 2 à 10 barg.

10. Procédé selon l'une quelconque des revendications précédentes, dans lequel un catalyseur de dépolymérisation frais est introduit dans le réacteur (4).

11. Procédé selon l'une quelconque des revendications précédentes, dans lequel le produit pyrolytique sous forme d'huile récupéré à partir de l'unité de condensation (5) présente la composition suivante (déterminée par CG) :
- environ 10 à 15 % en poids d'une fraction présentant un temps de rétention égal ou inférieur à celui du n-heptane ;
- environ 70 à 75 % en poids d'une fraction présentant un temps de rétention situé entre celui du n-heptane et celui du n-dodécane et
environ 12 à 20 % en poids de produit présentant un temps de rétention supérieur à celui du n-dodécane et inférieur à celui du n-octacosane.

12. Procédé selon l'une quelconque des revendications précédentes, dans lequel le produit pyrolytique sous forme d'huile récupéré à partir de la deuxième unité de condensation est utilisé en tant que charge hydrocarbonée dans des installations de craquage.

13. Procédé selon l'une quelconque des revendications précédentes, dans lequel au moins une partie de l'effluent liquide produit dans le premier réacteur (2) est guidée vers une section de manipulation de produit de carbonisation comprenant :
- une première chambre chemisée (6a), pourvue d'un conduit d'entrée (6a-1) destiné à introduire une suspension contenant du produit de carbonisation et d'un système d'agitation (6b), pouvant fonctionner à une température située dans la plage de 350 à 570 °C et pourvue en outre d'un conduit (6b-1) destiné à soutirer un effluent gazeux et
- des moyens (6c) destinés à soutirer une suspension concentrée à partir de la chambre (6a) et à l'acheminer vers une deuxième chambre (6d), lesdits moyens (6c) pouvant maintenir la suspension dans la même plage de température que celle de la chambre (6a) et étant pourvus d'un système de dégazage destiné à éliminer un effluent gazeux et à produire un produit de carbonisation drainé ;
- une deuxième chambre de désextraction (6d), recevant le produit de carbonisation drainé, équipée d'un système d'agitation (6d-1), d'une sortie de produit de carbonisation (6d-2), d'une entrée de gaz (6d-3) située dans le fond de ladite chambre et d'une sortie de gaz (6d-4) destinée à éliminer l'effluent gazeux extrait du produit de carbonisation ;
- des moyens (6e) destinés à recevoir le produit de carbonisation sec à partir de la chambre de désextraction (6d) et à l'acheminer vers une troisième chambre de collecte (6f), lesdits moyens (6e) pouvant maintenir le produit de carbonisation à une température située dans la plage de 60 à 100 °C ;
- une troisième chambre de collecte (6f) recevant le produit de carbonisation sec, pourvue d'un système d'agitation (6f-1), d'une sortie destinée à évacuer le produit de carbonisation, actionnée par une vanne (6f-2).

14. Procédé selon l'une quelconque des revendications 1 à 12, dans lequel au moins une partie de l'effluent liquide produit dans le premier réacteur (2) est guidée vers une section de manipulation de produit de carbonisation comprenant
- deux chambres chemisées (17, 18) ou plus, pourvues d'un conduit d'entrée (17a, 18a) destiné à introduire la suspension contenant le produit de carbonisation et d'un système d'agitation (17b, 18b), pouvant fonctionner à une température située dans la plage de 350 à 570 °C et pourvu en outre d'un conduit (17c ; 18c) destiné à soutirer un effluent gazeux et d'un conduit (17d, 18d) destiné à soutirer un produit de carbonisation séché à envoyer aux moyens d'acheminement ;
- des moyens (19, 20) destinés à acheminer le produit de carbonisation vers une chambre de collecte (21), lesdits moyens (19, 20) pouvant abaisser la température du produit de carbonisation sortant du couple de chambres chemisées (17, 18) ; et
- une chambre de collecte (21) recevant le produit de carbonisation sec, pourvue d'un système d'agitation (21a) et d'une sortie destinée à évacuer le produit de carbonisation.

15. Procédé selon l'une quelconque des revendications précédentes, dans lequel la teneur en produit de carbonisation dans l'effluent liquide produit dans le premier réacteur (2) sous la forme d'une suspension et guidé vers la section de manipulation de produit de carbonisation est située dans la plage de 10 à 50 %, de préférence de 20 à 40 % en poids, sur la base du poids total de la suspension.
